# EUROPEAN PATENT SPECIFICATION

(11) **EP 1 804 375 B1**
(45) Date of publication and mention of the grant of the patent: **08.10.2008**
(21) Application number: 06250897.3
(22) Date of filing: 20.02.2006
(51) Int. Cl.: H03F 3/45

(54) **Differential amplifier circuit operable with wide range of input voltages**
Differenzverstärker mit weitem Eingangsspannungsbereich
Amplificateur différentiel avec une gamme étendue de tentions d'entrée

(30) Priority: 27.12.2005 JP 2005375680
(43) Date of publication of application: 04.07.2007
(73) Proprietor: FUJITSU LIMITED, Kawasaki-shi, Kanagawa 211-8588 (JP)
(72) Inventor: Nakamoto, Junko, Kawasaki-shi Kanagawa 211-8588 (JP); Naka, Naoaki, Kawasaki-shi Kanagawa 211-8588 (JP)
(74) Representative: Hitching, Peter Matthew

(56) References cited:
- EP-A- 0 837 558
- US-A1- 2001 052 818
- US-A1- 2002 125 950

## Description

### BACKGROUND OF THE INVENTION

### 1. Field of the Invention

The present invention generally relates to amplifier circuits for amplifying signals, and particularly relates to a differential amplifier circuit for amplifying differential input signals.

### 2. Description of the Related Art

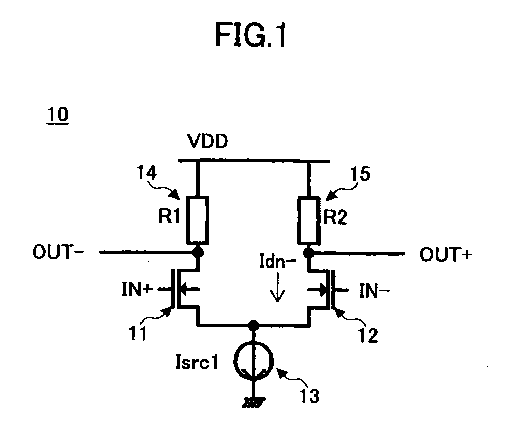

Fig. 1 is a drawing showing an example of the circuit configuration of a related-art differential amplifier circuit. Although this example shows a circuit configuration using NMOS transistors, a differential amplifier circuit may as well be implemented by use of PMOS transistors.

A differential amplifier circuit 10 shown in Fig. 1 includes an NMOS transistor 11, an NMOS transistor 12, a constant current source 13, a resistor 14, and a resistor 15. The gate node of the NMOS transistor 11 corresponds to an input node IN+, and the gate node of the NMOS transistor 12 corresponds to an input node IN-. A joint point between the drain node of the NMOS transistor 11 and the resistor 14 corresponds to an output node OUT-, and a joint point between the drain node of the NMOS transistor 12 and the resistor 15 corresponds to an output node OUT+. The amount of the current running through the constant current source 13 is denoted as Isrc1. The amount of the current running through the NMOS transistor 12 is denoted as Idn-.

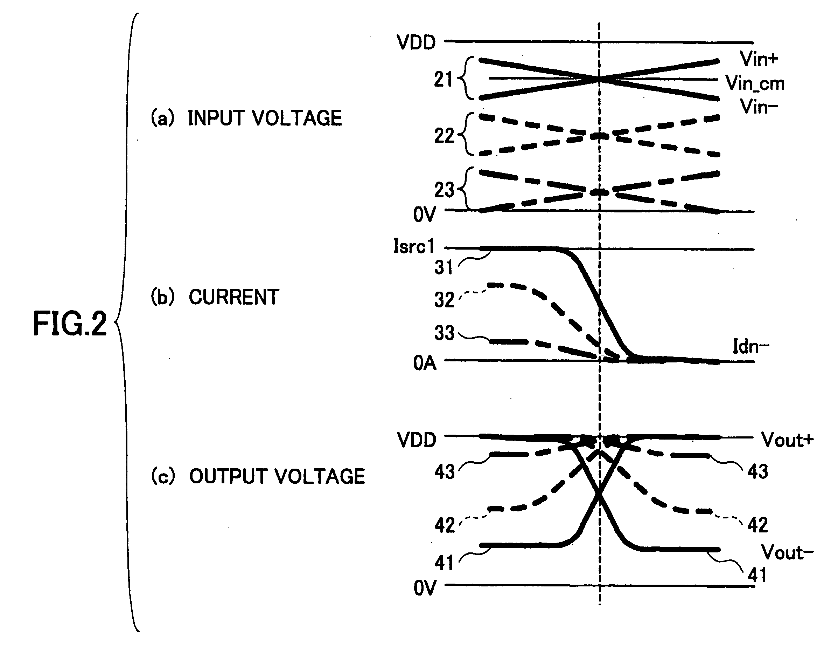

Fig. 2 is a drawing for explaining the operation of the differential amplifier circuit 10 shown in Fig. 1. A chart portion (a) illustrates input voltage waveforms that are input into the input nodes IN+ and IN-. A chart portion (b) illustrates the current Idn- flowing through the NMOS transistor 12. A chart portion (c) illustrates the output voltage waveforms that are output from the output nodes OUT+ and OUT-.

In Fig. 2-(a), a voltage waveform 21 shown by use of solid lines represents input voltages satisfying the input voltage conditions that are required in order for the differential amplifier circuit 10 to operate properly. The voltage applied to the input node IN+ is shown as Vin+, and the voltage applied to the input node IN- is shown as Vin-. Here, Vin_cm represents an input common-mode voltage, which is equal to an average of Vin+ and Vin-. In Fig. 2-(a), the voltage Vin+ rises and the voltage Vin- falls from left to right in the drawing (e.g., as time passes).

In Fig. 2-(b), a current waveform 31 shown by use of a solid curved line represents changes in the current Idn- when the input voltages having the voltage waveform 21 is applied. In Fig. 1, as the voltage Vin+ applied to the input node IN+ rises, the conductivity of the NMOS transistor 11 increases. As the voltage Vin- applied to the input node IN-falls, the conductivity of the NMOS transistor 12 decreases. Assuming that the current Isrc1 running through the constant current source 13 is constant, an increase in the current flowing through the NMOS transistor 11 results in the current flowing through the NMOS transistor 12 decreasing by an amount commensurate with such an increase. This reduction of the current Idn- running through the NMOS transistor 12 is shown as the current waveform 31 in Fig. 2-(b).

In Fig. 2-(c), a voltage waveform 41 shown by use of a solid curved line represents changes in the output voltages when the input voltages having the voltage waveform 21 is applied. The voltage output from the output node OUT+ is shown as Vout+, and the voltage output from the output node OUT- is shown as Vout-. As the current running through the NMOS transistor 11 increases, a voltage drop across the resistor 14 conducting this current increases, resulting in a drop in the output voltage Vout-. As the current running through the NMOS transistor 12 decreases, a voltage drop across the resistor 15 conducting this current decreases, resulting in a rise in the output voltage Vout+. The amounts of the changes of the output voltages Vout- and Vout+ are proportional to the respective resistances R1 and R2 of the resistors 14 and 15, respectively. The larger the resistances R1 and R2, the greater the amplification factor is.

In Fig. 2-(a), a voltage waveform 22 shown by use of dotted lines represents a case in which the input voltages Vin+ and Vin- are both lowered compared with the voltage waveform 21. In this case, as shown by a current waveform 32 illustrated by use of a dotted curved line in (b), the amount of a change in the current Idn- becomes smaller than that of the current waveform 31. In response, as shown by a voltage waveform 42 illustrated by use of dotted lines in (c), the amounts of changes in the output voltages Vout- and the output voltage Vout+ become smaller than those of the voltage waveform 41. Namely, the amplification factor falls.

In Fig. 2-(a), a voltage waveform 23 shown by use of chain lines represents a case in which the input voltages Vin+ and Vin- are both lowered further. Here, the potential at the source node of the NMOS transistors 11 and 12 is referred to as Vn1, and the threshold voltage of an NMOS transistor is denoted as Vth. If the input voltages Vin+ and Vin- become substantially comparable to or lower than Vn1+Vth, the differential amplifier circuit 10 almost stops performing proper amplification. Namely, as shown by a current waveform 33 illustrated by use of a chain curved line in (b), the current Idn- ends up showing almost no changes. In response, as shown by a voltage waveform 43 illustrated by use of chain lines in (c), there are almost no changes in the output voltages Vout- and the output voltage Vout+, resulting in the amplification operation of the differential amplifier circuit 10 being undermined.

If the input voltages Vin+ and Vin- are lowered fully below Vn1+Vth, changes in the output voltages Vout- and the output voltage Vout+ disappear substantially, resulting in the amplification operation of the differential amplifier circuit 10 being completely undermined. Namely, the differential amplifier circuit 10 has an insensitive area that is equal in size to the threshold voltage Vth with respect to the input voltages, so that the range of input voltages required for proper amplification operation is limited by this insensitive area.

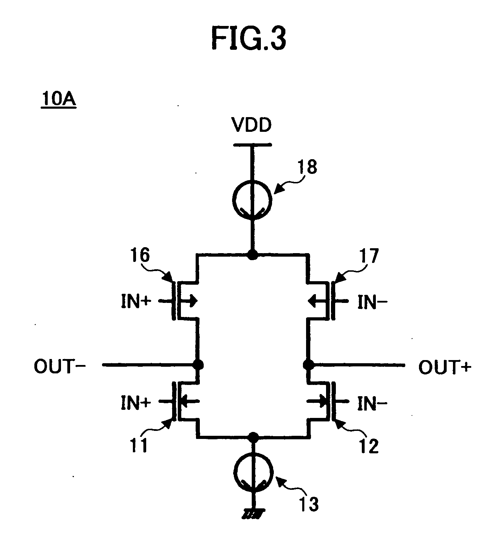

Fig. 3 is a drawing showing another example of the circuit configuration of a related-art differential amplifier circuit. This circuit is configured with an aim to obviate the problem of the insensitive area corresponding to the threshold voltage Vth. In Fig. 3, the same elements as those of Fig. 1 are referred to by the same numerals, and a description thereof will be omitted.

A differential amplifier circuit 10A shown in Fig. 3 includes an NMOS transistor 11, an NMOS transistor 12, a constant current source 13, a PMOS transistor 16, a PMOS transistor 17, and a constant current source 18. In this differential amplifier circuit 10A, the gate node of the PMOS transistor 16 is the input node IN+ that is the same as the gate node of the NMOS transistor 11, and receives the same input voltage Vin+. The gate node of the PMOS transistor 17 is the input node IN- that is the same as the gate node of the NMOS transistor 12, and receives the same input voltage Vin-. The PMOS transistor 16, the PMOS transistor 17, and the constant current source 18 together constitute a P-channel differential amplifier circuit, which performs an operation similar to the operation of the N-channel differential amplifier circuit comprised of the NMOS transistor 11, the NMOS transistor 12, and the constant current source 13.

In this configuration, even if the input voltages Vin+ and Vin- are lowered, a sufficiently large gate-source voltage is applied to the PMOS transistors 16 and 17, so that the P-channel differential amplifier circuit performs proper amplification operation. As a result, even if the input voltage conditions are such that the N-channel differential amplifier circuit cannot perform a proper amplification operation, the combination of the N-channel side and the P-channel side as a whole can provide a proper amplification operation. Here, if the input voltages Vin+ and Vin- are high (as in the case of the input voltage waveform 21 shown in Fig. 2-(a)), a sufficiently large gate-source voltage cannot be maintained for the PMOS transistors 16 and 17, so that the P-channel differential amplifier circuit cannot perform proper amplification operation. In such a case, however, the N-channel differential amplifier circuit performs a proper amplification operation, so that the combination of the N-channel side and the P-channel side as a whole can provide a proper amplification operation.

In the case of the circuit configuration shown in Fig. 3, the two constant current sources, one PMOS transistor, and one NMOS transistor are stacked one over the other to form multiple stages between the power supply potential VDD and the ground potential GND. The number of stacked stages is four. This is one stage more than the three stacked stages of the circuit configuration shown in Fig. 1.

If the power supply voltage VDD falls for some reason, resulting in a situation in which a sufficient voltage is not applied to each device, then, a proper operation is lost regardless of the circuit configuration. The circuit configuration shown in Fig. 1 has three stacked stages, so that the differential amplifier circuit 10 properly operates when the power supply voltage VDD is at least three times as high as the voltage required for one device to properly operate. Even with this particular power supply voltage VDD that allows the differential amplifier circuit 10 to properly operate, the differential amplifier circuit 10A of Fig. 3 cannot operate properly since this circuit requires a power supply voltage four times as high as the voltage required for one device to properly operate. Namely, the differential amplifier circuit 10A shown in Fig. 3 is more susceptible to drop in the power supply voltage VDD than the differential amplifier circuit 10 shown in Fig. 1.

Patent Document 1 discloses a CMOS operational amplifier circuit that can properly operate with respect to a wide range of input/output voltages, and that can perform highly accurate amplification, serving as a differential amplifier circuit having a similar structure to that of the circuit shown in Fig. 3.

[Patent Document 1] Japanese Patent Application Publication No. 2002-344261

Patent application publication US 2001/0052818 A1 discloses a differential amplifier with rail-to-rail input complementary input stages. The first input stage consists of a NMOS differential pair having a tail current connected between the GND potential and the common source node of the pair. The second input stage consists of a PMOS differential pair having a tail current connected between the positive power supply and the common source node of the pair. The differential drain currents of the differential pairs are summed together and delivered to the output stage of the amplifier.

Accordingly, there is a need for a differential amplifier circuit that can properly operate with respect to a wide range of input voltages, and that can properly operate with a low power supply voltage.

### SUMMARY OF THE INVENTION

It is a general object of the present invention to provide a differential amplifier circuit that substantially obviates one or more problems caused by the limitations and disadvantages of the related art.

Features and advantages of the present invention will be presented in the description which follows, and in part will become apparent from the description and the accompanying drawings, or may be learned by practice of the invention according to the teachings provided in the description. Objects as well as other features and advantages of the present invention will be realized and attained by a differential amplifier circuit particularly pointed out in the specification in such full, clear, concise, and exact terms as to enable a person having ordinary skill in the art to practice the invention.

To achieve these and other advantages in accordance with the purpose of the invention, the invention provides a differential amplifier circuit which includes a first load having an end thereof coupled to a first reference potential, a first MOS transistor having a drain node thereof coupled to another node of the first load, a second load having an end thereof coupled to the first reference potential, a second MOS transistor having a drain node thereof coupled to another node of the second load, a first constant current source coupled between a second reference potential and both a source node of the first MOS transistor and a source node of the second MOS transistor, a third MOS transistor having a source node thereof coupled to said another node of the first load, a fourth MOS transistor having a source node thereof coupled to said another node of the second load, and a second constant current source coupled between the second reference potential and both a drain node of the third MOS transistor and a drain node of the fourth MOS transistor, wherein the first and fourth MOS transistors have gate nodes thereof coupled to each other, and the second and third MOS transistors have gate nodes thereof coupled to each other, the first and second MOS transistors being of a first conduction type, and the third and fourth MOS transistors being of a second conduction type.

According to at least one embodiment of the present invention, the differential amplifier circuit is configured such that a circuit portion comprised of MOS transistors of a first conduction type and a circuit portion comprised of MOS transistors of a second conduction type are provided in parallel, so that at least one of the circuit portions can properly operate regardless of high/low of the input voltages. Because of this, there is no insensitive area that is equal in size to the threshold voltage Vth with respect to the input voltages, so that a proper operation is achievable with respect to a wide range of input voltages. With the number of multiple stacked stages being three, the differential amplifier circuit can properly operate when the power supply voltage is at least three times as high as the voltage required for one device to properly operate.

### BRIEF DESCRIPTION OF THE DRAWINGS

Other objects and further features of the present invention will be apparent from the following detailed description when read in conjunction with the accompanying drawings, in which:
Fig. 1 is a drawing showing an example of the circuit configuration of a related-art differential amplifier circuit;
Fig. 2 is a drawing for explaining the operation of the differential amplifier circuit shown in Fig. 1;
Fig. 3 is a drawing showing another example of the circuit configuration of a related-art differential amplifier circuit;
Fig. 4 is a drawing showing the circuit configuration of a first embodiment of a differential amplifier circuit according to the present invention;
Fig. 5 is a drawing for explaining the operation of the differential amplifier circuit shown in Fig. 4;
Fig. 6 is a drawing showing the circuit configuration of a second embodiment of the differential amplifier circuit according to the present invention;
Fig. 7 is a drawing showing the circuit configuration of a third embodiment of the differential amplifier circuit according to the present invention;
Fig. 8 is a drawing showing the circuit configuration of a fourth embodiment of the differential amplifier circuit according to the present invention; and
Fig. 9 is a drawing showing the circuit configuration of a fifth embodiment of the differential amplifier circuit according to the present invention.

### DESCRIPTION OF THE PREFERRED EMBODIMENTS

In the following, embodiments of the present invention will be described with reference to the accompanying drawings.

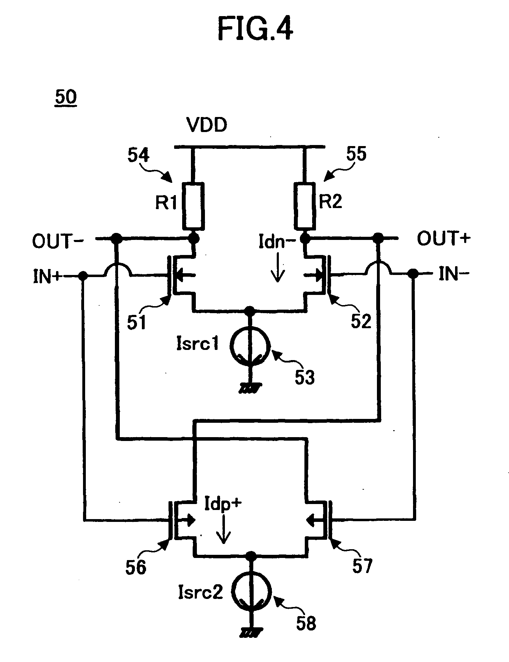

Fig. 4 is a drawing showing the circuit configuration of a first embodiment of a differential amplifier circuit according to the present invention. A differential amplifier circuit 50 shown in Fig. 4 includes an NMOS transistor 51, an NMOS transistor 52, a constant current source 53, a resistor 54, a resistor 55, a PMOS transistor 56, a PMOS transistor 57, and a constant current source 58.

The resistor 54, the NMOS transistor 51, and the constant current source 53 are connected in series in the order named between the power supply voltage VDD and the ground voltage. Further, sharing the constant current source 53 with this series connection, the resistor 55, the NMOS transistor 52, and the constant current source 53 are connected in series in the order named between the power supply voltage VDD and the ground voltage.

The gate node of the NMOS transistor 51 serves as an input node IN+, and the gate node of the NMOS transistor 52 serves as an input node IN-. A joint point between the drain node of the NMOS transistor 51 and the resistor 54 serves as an output node OUT-, and a joint point between the drain node of the NMOS transistor 52 and the resistor 55 serves as an output node OUT+. The amount of the current running through the constant current source 53 is denoted as Isrc1. The amount of the current running through the NMOS transistor 52 is denoted as Idn-.

The PMOS transistor 57 and the constant current source 58 are connected in series in the order named between the output node OUT- (i.e., the joint point between the drain node of the NMOS transistor 51 and the resistor 54) and the ground potential. Further, sharing the constant current source 58 with this series connection, the PMOS transistor 56 and the constant current source 58 are connected in series in the order named between the output node OUT+ (i.e., the joint point between the drain node of the NMOS transistor 52 and the resistor 55) and the ground potential. The amount of the current running through the constant current source 58 is denoted as Isrc2. The amount of the current running through the PMOS transistor 56 is denoted as Idp+.

The gate node of the PMOS transistor 56 also serves as the input node IN+, and the gate node of the PMOS transistor 57 also serves as the input node IN-. Namely, the gate node of the NMOS transistor 51 and the gate node of the PMOS transistor 56 are connected to the same input node IN+, and the gate node of the NMOS transistor 52 and the gate node of the PMOS transistor 57 are connected to the same input node IN-.

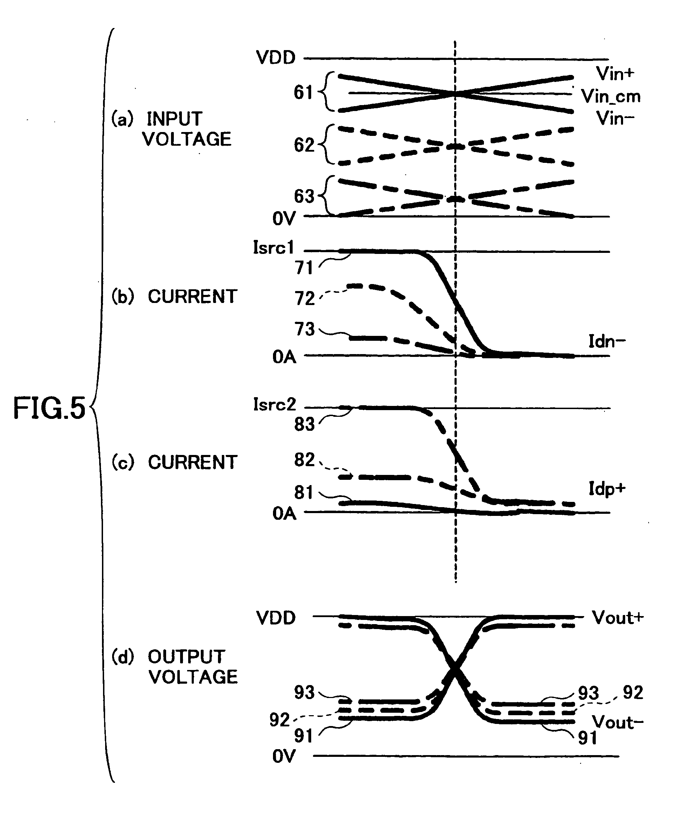

Fig. 5 is a drawing for explaining the operation of the differential amplifier circuit 50 shown in Fig. 4. A chart portion (a) illustrates input voltage waveforms that are input into the input nodes IN+ and IN-. A chart portion (b) illustrates the current Idn- flowing through the NMOS transistor 52. A chart portion (c) illustrates the current Idp+ running through the PMOS transistor 56. A chart portion (d) illustrates the output voltage waveforms that are output from the output nodes OUT+ and OUT-.

In Fig. 5-(a), a voltage waveform 61 shown by use of solid lines represents input voltages satisfying the input voltage conditions in which input voltages falling within a range close to the power supply voltage are provided. The voltage applied to the input node IN+ is shown as Vin+, and the voltage applied to the input node IN- is shown as Vin-. Here, Vin_cm represents an input common-mode voltage, which is equal to an average of Vin+ and Vin-. In Fig. 5-(a), the voltage Vin+ rises and the voltage Vin- falls from left to right in the drawing (e.g., as time passes).

In Fig. 5-(b), a current waveform 71 shown by use of a solid curved line represents changes in the current Idn- when the input voltages having the voltage waveform 61 is applied. In Fig. 4, as the voltage Vin+ applied to the input node IN+ rises, the conductivity of the NMOS transistor 51 increases. As the voltage Vin- applied to the input node IN-falls, the conductivity of the NMOS transistor 52 decreases. Assuming that the current Isrc1 running through the constant current source 53 is constant, an increase in the current flowing through the NMOS transistor 51 results in the current flowing through the NMOS transistor 52 decreasing by an amount commensurate with such an increase. This reduction of the current Idn- running through the NMOS transistor 52 is shown as the current waveform 71 in Fig. 5-(b).

In Fig. 5-(c), a current waveform 81 shown by use of a solid curved line represents changes in the current Idp+ when the input voltages having the voltage waveform 61 is applied. In Fig. 4, as the voltage Vin+ applied to the input node IN+ rises, the conductivity of the PMOS transistor 56 increases. As the voltage Vin- applied to the input node IN-falls, the conductivity of the PMOS transistor 57 decreases. Assuming that the current Isrc2 running through the constant current source 58 is constant, a decrease in the current flowing through the PMOS transistor 56 results in the current flowing through the PMOS transistor 57 increasing by an amount commensurate with such a decrease. This reduction of the current Idp+ running through the PMOS transistor 56 is shown as the current waveform 81 in Fig. 5-(c).

In this case, however, the input voltages shown as the voltage waveform 61 (Fig. 5-(a)) are close to the power supply voltage VDD, so that the PMOS transistors 56 and 57 become conductive only slightly. Further, under the condition in which the PMOS transistor 56 becomes conductive, i.e., when the voltage Vin+ applied to the input node IN+ is low, the voltage Vin- applied to the input node IN-is relatively high, so that the NMOS transistor 52 becomes conductive to reduce the voltage at the output node OUT+. Because of this, the voltage that sufficiently exceeds the threshold voltage of a transistor is not applied between the source node (OUT+) and gate node (IN+) of the PMOS transistor 56, so that the amount of the current Idp+ is extremely small as shown in the current waveform 81.

In Fig. 5-(d), a voltage waveform 91 shown by use of solid curved lines represents changes in the output voltages when the input voltages having the voltage waveform 61 is applied. The voltage output from the output node OUT+ is shown as Vout+, and the voltage output from the output node OUT- is shown as Vout-. As the current running through the NMOS transistor 51 increases, a voltage drop across the resistor 54 conducting this current increases, resulting in a drop in the output voltage Vout-. As the current running through the NMOS transistor 52 decreases, a voltage drop across the resistor 55 conducting this current decreases, resulting in a rise in the output voltage Vout+. The amounts of the changes of the output voltages Vout- and Vout+ are proportional to the respective resistances R1 and R2 of the resistors 54 and 55, respectively. The larger the resistances R1 and R2, the greater the amplification factor is.

In Fig. 5-(a), a voltage waveform 62 shown by use of dotted lines represents a case in which the input voltages Vin+ and Vin- are both lowered compared with the voltage waveform 61. In this case, as shown by a current waveform 72 illustrated by use of a dotted curved line in (b), the amount of a change in the current Idn- becomes smaller than that of the current waveform 71. Conversely, as shown by a current waveform 82 illustrated by use of a dotted curved line in (c), the amount of a change in the current Idp+ becomes larger than that of the current waveform 81. An increase in the change of the current Idp+, however, is smaller than a decrease in the change of the current Idn-. In response, as shown by a voltage waveform 92 illustrated by use of dotted lines in (d), the amounts of changes in the output voltages Vout- and the output voltage Vout+ become slightly smaller than those of the voltage waveform 91. However, because of the effect of an increase in the change of the current Idp+, the amplification factor does not decrease so much as in the case of the output voltage waveform 42 shown in Fig. 2-(c).

In Fig. 5-(a), a voltage waveform 63 shown by use of chain lines represents a case in which the input voltages Vin+ and Vin- are both lowered further. Here, the potential at the source node of the NMOS transistors 51 and 52 is referred to as Vn1, and the threshold voltage of an NMOS transistor is denoted as Vth. If the input voltages Vin+ and Vin-become substantially comparable to or lower than Vn1+Vth, the N-channel-based circuit of the differential amplifier circuit 50 almost stops performing proper amplification. Namely, as shown by a current waveform 73 illustrated by use of a chain curved line in (b), the current Idn- ends up showing almost no changes.

Due to the fact that the input voltages Vin+ and Vin- are significantly low, one of the PMOS transistor 56 and the PMOS transistor 57 that is supposed to be conductive becomes conductive sufficiently. Accordingly, as shown by a current waveform 83 illustrated by use of a chain line in (c), the current Idp+ changes fully in the range from zero to the current amount Isrc2.

In response, as shown by a voltage waveform 93 illustrated by use of chain lines in (d), the amplification operation of the differential amplifier circuit is not lost even though the amplification factor is slightly lowered compared with the case of the voltage waveform 91. That is, proper amplification operation is maintained. When the input voltages are lowered, the N-channel-based differential amplifier comprised of the NMOS transistor 51, the NMOS transistor 52, and the constant current source 53 loses its proper amplification operation. Nonetheless, the P-channel-based circuit comprised of the PMOS transistor 56, the PMOS transistor 57, and the constant current source 58 properly operates, so that the differential amplifier circuit 50 as a whole can provide a proper amplification operation.

Even if the input voltages Vin+ and Vin-are lowered fully below Vn1+Vth, changes in the output voltages Vout- and the output voltage Vout+ do not disappear. Namely, the differential amplifier circuit 50 does not have the insensitive area that is equal in size to the threshold voltage Vth with respect to the input voltages, so that the range of input voltages required for proper amplification operation is not limited by this insensitive area.

In the differential amplifier circuit 50 shown in Fig. 4, the output voltage Vout+ is equal to Vdd-R2{(Idn-)+(Idp+)}. As Vout+ rises, the conductivity of the PMOS transistor 56 increases, which serves to pull down the level of the voltage Vout+. Accordingly, the amplification factor of the differential amplifier circuit 50 becomes slightly smaller than the amplification factor of the differential amplifier circuit 10 shown in Fig. 1. However, the effect of suppressing the fluctuation of the output voltage levels responsive to the fluctuation of the input voltage levels is obtained.

Further, provided that the constant current sources 53 and 58 have the same current amount (Isrc1=Isrc2), the same output voltage levels are maintained between when the N-channel-based circuit of the differential amplifier circuit 50 operates with the P-channel-based circuit almost failing to operate and when the P-channel-based circuit of the differential amplifier circuit 50 operates with the N-channel-based circuit almost failing to operate. Namely, the voltage Vout+ of the output node OUT+ or the voltage Vout- of the output node OUT-, whichever is higher, can be kept constant regardless of how high/low the input voltages are.

In the differential amplifier circuit 50 shown in Fig. 4, further, one resistor (54 or 55), one transistor (51, 52, 56, or 57), and one constant current source (53 or 58) are provided between the power supply potential VDD and the ground potential. That is, the number of stacked stages is three. This number of stages is smaller than that of the related-art circuit configuration shown in Fig. 3. Even with a low voltage that does not allow the circuit of Fig. 3 to properly operate, therefore, the differential amplifier circuit 50 can properly operate if the power supply voltage VDD is at least three times as high as the voltage required for one device to properly operate.

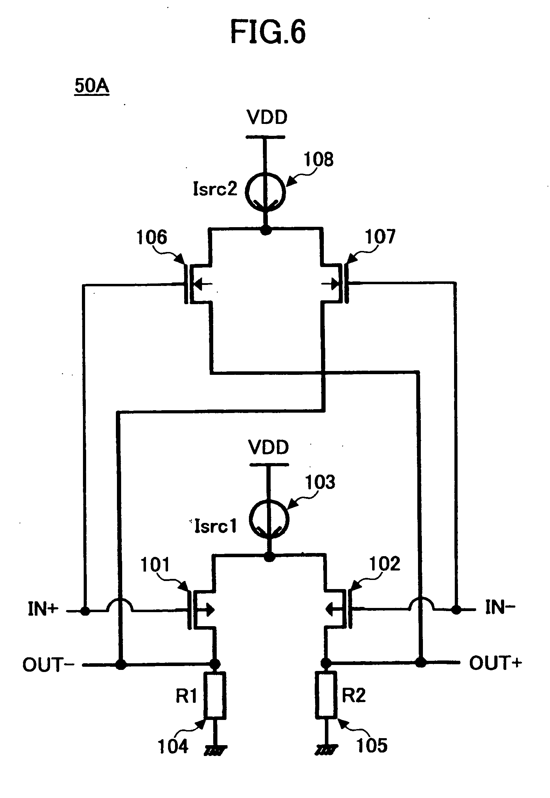

Fig. 6 is a drawing showing the circuit configuration of a second embodiment of a differential amplifier circuit according to the present invention. A differential amplifier circuit 50A shown in Fig. 6 includes a PMOS transistor 101, a PMOS transistor 102, a constant current source 103, a resistor 104, a resistor 105, an NMOS transistor 106, an NMOS transistor 107, and a constant current source 108.

The resistor 104, the PMOS transistor 101, and the constant current source 103 are connected in series between the ground voltage and the power supply voltage VDD. Further, sharing the constant current source 103 with this series connection, the resistor 105, the PMOS transistor 102, and the constant current source 103 are connected in series between the ground voltage and the power supply voltage VDD.

The gate node of the PMOS transistor 101 serves as an input node IN+, and the gate node of the PMOS transistor 102 serves as an input node IN-. A joint point between the drain node of the PMOS transistor 101 and the resistor 104 serves as an output node OUT-, and a joint point between the drain node of the PMOS transistor 102 and the resistor 105 serves as an output node OUT+.

The NMOS transistor 107 and the constant current source 108 are connected in series between the output node OUT- (i.e., the joint point between the drain node of the PMOS transistor 101 and the resistor 104) and the power supply potential VDD. Further, sharing the constant current source 108 with this series connection, the NMOS transistor 106 and the constant current source 108 are connected in series between the output node OUT+ (i.e., the joint point between the drain node of the PMOS transistor 102 and the resistor 105) and the power supply potential VDD.

The gate node of the NMOS transistor 106 also serves as the input node IN+, and the gate node of the NMOS transistor 107 also serves as the input node IN-. Namely, the gate node of the PMOS transistor 101 and the gate node of the NMOS transistor 106 are connected to the same input node IN+, and the gate node of the PMOS transistor 102 and the gate node of the NMOS transistor 107 are connected to the same input node IN-.

In the differential amplifier circuit 50A shown in Fig. 6, NMOS and PMOS are swapped compared with the differential amplifier circuit 50 shown in Fig. 4. The differential amplifier circuit 50A having such configuration operates in the same manner as the differential amplifier circuit 50, except that the role of the N-channel side and the role of the P-channel side are swapped, thereby bringing about the same effects and advantages. Namely, the differential amplifier circuit 50A does not have the insensitive area that is equal in size to the threshold voltage Vth with respect to the input voltages, so that the range of input voltages required for proper amplification operation is not limited by the presence of such insensitive area.

In the differential amplifier circuit 50A shown in Fig. 4, the number of multiple stacked stages is three. Even with a low power supply voltage that does not allow the related-art circuit of Fig. 3 to properly operate, the differential amplifier circuit 50A can properly operate if the power supply voltage VDD is at least three times as high as the voltage required for one device to properly operate.

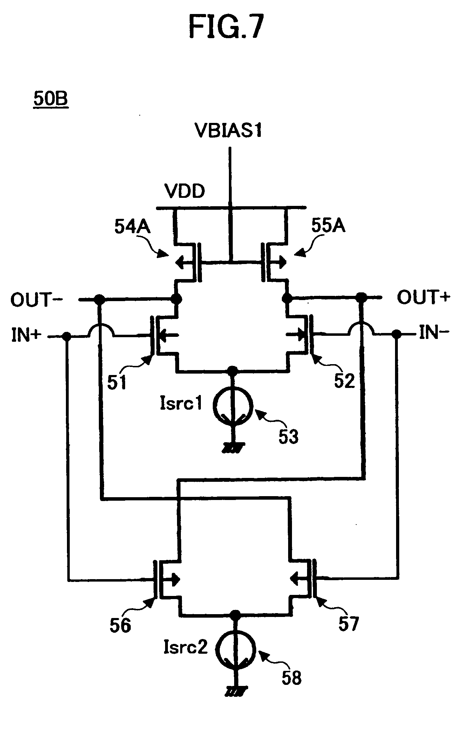

Fig. 7 is a drawing showing the circuit configuration of a third embodiment of a differential amplifier circuit according to the present invention. In Fig. 7, the same elements as those of Fig. 4 are referred to by the same numerals, and a description thereof will be omitted.

In a differential amplifier circuit 50B shown in Fig. 7, the resistors 54 and 55 of the differential amplifier circuit 50 shown in Fig. 4 are replaced with PMOS transistors 54A and 55A. Other parts of the configuration are the same between Fig. 7 and Fig. 4. The gate nodes of the PMOS transistors 54A and 55A receive a common bias voltage VBIAS.

Since the source-gate voltage of the PMOS transistors 54A and 55A is constant, the source-drain voltage can be changed significantly with little change in the drain currents Namely, the PMOS transistors 54A and 55A can serve as a resistor having an extremely large resistance. In the configuration shown in Fig. 7, the gate nodes of the PMOS transistors 54A and 55A receive the common bias voltage VBIAS1, so that the amplification factor of the differential amplifier circuit 50B can be easily controlled by adjusting the bias voltage VBIAS1.

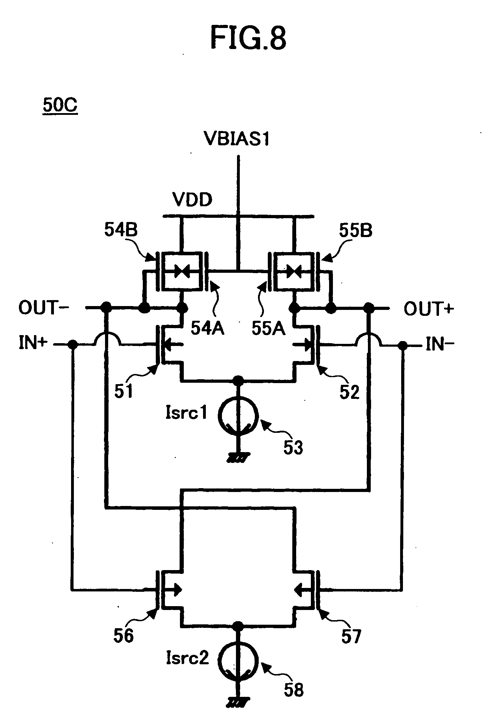

Fig. 8 is a drawing showing the circuit configuration of a fourth embodiment of a differential amplifier circuit according to the present invention. In Fig. 8, the same elements as those of Fig. 7 are referred to by the same numerals, and a description thereof will be omitted.

In a differential amplifier circuit 50C shown in Fig. 8, PMOS transistors 54B and 55B are connected in parallel to the PMOS transistors 54A and 55A, respectively, of the differential amplifier circuit 50B shown in Fig. 7. Other parts of the configuration are the same between Fig. 8 and Fig. 7. The gate node of the PMOS transistor 54B is connected to the output node OUT-, and the gate node of the PMOS transistor 55B is connected to the output node OUT+.

The PMOS transistors 54A and 55A serve as a resistor having an extremely large resistance, so that the amplification factor of the differential amplifier circuit 50C can be easily controlled by adjusting the bias voltage VBIAS1. Further, as Vout+ rises, the conductivity of the PMOS transistor 55B decreases, which serves to pull down the level of the voltage Vout+. The relationship between Vout-and the PMOS transistor 54B is also the same. Accordingly, the PMOS transistors 54B and 55B serve to suppress the amplification factor of the differential amplifier circuit 50C. With this provision, the operation of the differential amplifier circuit 50C can be further stabilized.

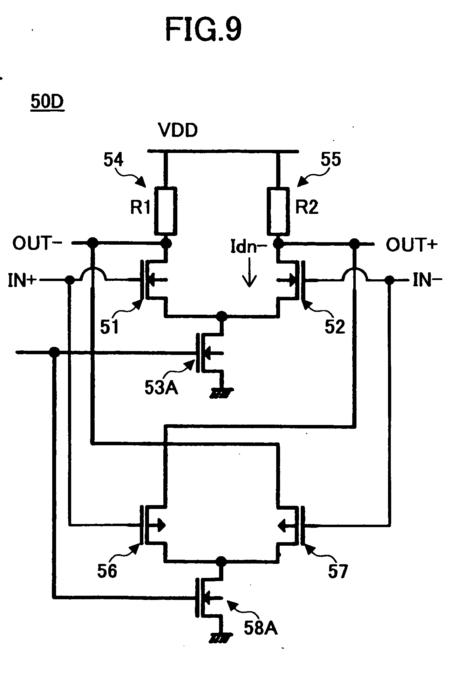

Fig. 9 is a drawing showing the circuit configuration of a fifth embodiment of a differential amplifier circuit according to the present invention. In Fig. 9, the same elements as those of Fig. 4 are referred to by the same numerals, and a description thereof will be omitted.

In a differential amplifier circuit 50D shown in Fig. 9, the constant current sources 53 and 58 of the differential amplifier circuit 50 shown in Fig. 4 are replaced with NMOS transistors 53A and 58A. Other parts of the configuration are the same between Fig. 9 and Fig. 4. The gate nodes of the NMOS transistors 53A and 58A receive a common bias voltage VBIAS2.

Since the source-gate voltage of the PMOS transistors 53A and 58A is constant, the PMOS transistors 53A and 58A can serve as a constant current source conducting a substantially constant current. Further, the amplification factor of the differential amplifier circuit 50D can be easily controlled by adjusting the bias voltage VBIAS2.

Moreover, the gate nodes of the NMOS transistors 53A and 58A may be set to a common bias voltage. With such provision, it is possible to set the amount of the current running through the NMOS transistor 53A and the amount of the current running through the NMOS transistor 58A substantially equal to the same amount. Namely, the same output voltage levels are maintained between when the N-channel-based circuit of the differential amplifier circuit 50D operates with the P-channel-based circuit almost failing to operate and when the P-channel-based circuit of the differential amplifier circuit 50D operates with the N-channel-based circuit almost failing to operate. Namely, the output voltage of the output node OUT+ or the output voltage of the output node OUT-, whichever is higher, can be kept constant regardless of how high/low the input voltages are.

Further, the present invention is not limited to these embodiments, but various variations and modifications may be made without departing from the scope of the present invention.

## Claims

1. A differential amplifier circuit, **characterized by** comprising:
a first load (54) having an end thereof coupled to a first reference potential (VDD);
a first MOS transistor (51) having a drain node thereof coupled to another node (OUT-) of the first load;
a second load (55) having an end thereof coupled to the first reference potential;
a second MOS transistor (52) having a drain node thereof coupled to another node (OUT+) of the second load;
a first constant current source (53) coupled between a second reference potential and both a source node of the first MOS transistor and a source node of the second MOS transistor;
a third MOS transistor (57) having a source node thereof coupled to said another node of the first load;
a fourth MOS transistor (56) having a source node thereof coupled to said another node of the second load; and
a second constant current source (58) coupled between the second reference potential and both a drain node of the third MOS transistor and a drain node of the fourth MOS transistor,
wherein the first and fourth MOS transistors have gate nodes thereof coupled to each other, and the second and third MOS transistors have gate nodes thereof coupled to each other, the first and second MOS transistors being of a first conduction type, and the third and fourth MOS transistors being of a second conduction type.

2. The differential amplifier circuit as claimed in claim 1, wherein the first and second MOS transistors are N-channel transistors, and the third and fourth MOS transistors are P-channel transistors.

3. The differential amplifier circuit as claimed in claim 1, wherein the first and second MOS transistors are P-channel transistors, and the third and fourth MOS transistors are N-channel transistors.

4. The differential amplifier circuit as claimed in claim 1, wherein the first load is a fifth MOS transistor (54A),and the second load is a sixth MOS transistors (55A).

5. The differential amplifier circuit as claimed in claim 4, wherein a gate node of the fifth MOS transistor and a gate node of the sixth MOS transistor are coupled to a common bias potential (VBIAS1).

6. The differential amplifier circuit as claimed in claim 1, wherein the first constant current source is a seventh MOS transistor (53A), and the second constant current source is an eighth MOS transistor (58A).

7. The differential amplifier circuit as claimed in claim 6, wherein a gate node of the seventh MOS transistor and a gate node of the eighth MOS transistor are coupled to a common bias potential.

## Patentansprüche

1. Differenzverstärkerschaltung, **dadurch gekennzeichnet, dass** sie umfasst:
eine erste Last (54), von der ein Ende mit einem ersten Referenzpotential (VDD) gekoppelt ist;
einen ersten MOS-Transistor (51), von dem ein Drain-Knoten mit einem anderen Knoten (OUT-) der ersten Last gekoppelt ist;
eine zweite Last (55), von der ein Ende mit dem ersten Referenzpotential gekoppelt ist;
einen zweiten MOS-Transistor (52), von dem ein Drain-Knoten mit einem anderen Knoten (OUT+) der zweiten Last gekoppelt ist;
eine erste Konstantstromquelle (59), die zwischen einem zweiten Referenzpotential und sowohl einem Source-Knoten des ersten MOS-Transistors als auch einem Source-Knoten des zweiten MOS-Transistors gekoppelt ist;
einen dritten MOS-Transistor (57), von dem ein Source-Knoten mit dem anderen Knoten der ersten Last gekoppelt ist;
einen vierten MOS-Transistor (56), von dem ein Source-Knoten mit dem anderen Knoten der zweiten Last gekoppelt ist; und
eine zweite Konstantstromquelle (58), die zwischen dem zweiten Referenzpotential und sowohl einem Drain-Knoten des dritten MOS-Transistors als auch einem Drain-Knoten des vierten MOS-Transistors gekoppelt ist,
bei der die ersten und vierten MOS-Transistoren Gate-Knoten haben, die miteinander gekoppelt sind, und die zweiten und dritten MOS-Transistoren Gate-Knoten haben, die miteinander gekoppelt sind, wobei die ersten und zweiten MOS-Transistoren von einem ersten Leitungstyp sind und die dritten und vierten MOS-Transistoren von einem zweiten Leitungstyp sind.

2. Differenzverstärkerschaltung nach Anspruch 1, bei der die ersten und zweiten MOS-Transistoren N-Kanal-Transistoren sind und die dritten und vierten MOS-Transistoren P-Kanal-Transistoren sind.

3. Differenzverstärkerschaltung nach Anspruch 1, bei der die ersten und zweiten MOS-Transistoren P-Kanal-Transistoren sind und die dritten und vierten MOS-Transistoren N-Kanal-Transistoren sind.

4. Differenzverstärkerschaltung nach Anspruch 1, bei der die erste Last ein fünfter MOS-Transistor (54A) ist und die zweite Last ein sechster MOS-Transistor (55A) ist.

5. Differenzverstärkerschaltung nach Anspruch 4, bei der ein Gate-Knoten des fünften MOS-Transistors und ein Gate-Knoten des sechsten MOS-Transistors mit einem gemeinsamen Vorspannungspotential (VBIAS1) gekoppelt sind.

6. Differenzverstärkerschaltung nach Anspruch 1, bei der die erste Konstantstromquelle ein siebter MOS-Transistor (53A) ist und die zweite Konstantstromquelle ein achter MOS-Transistor (58A) ist.

7. Differenzverstärkerschaltung nach Anspruch 6, bei der ein Gate-Knoten des siebten MOS-Transistors und ein Gate-Knoten des achten MOS-Transistors mit einem gemeinsamen Vorspannungspotential gekoppelt sind.

## Revendications

1. Amplificateur différentiel, **caractérisé en ce qu'**il comporte :
une première charge (54) présentant une de ses extrémités couplée à un premier potentiel de référence (VDD);
un premier transistor MOS (51) possédant un de ses noeuds de drain couplé à un autre noeud (OUT-) de la première charge ;
une seconde charge (55) possédant une de ses extrémités couplée au premier potentiel de référence ;
un second transistor MOS (52) possédant un de ses noeuds de drain couplé à un autre noeud (OUT+) de la seconde charge ;
une première source de courant constant (53) couplée entre un second potentiel de référence et à la fois un noeud de source du premier transistor MOS et un noeud de source du second transistor MOS ;
un troisième transistor MOS (57) possédant un de ses noeuds de source couplé audit autre noeud de la première charge ;
un quatrième transistor MOS (56) possédant un de ses noeuds de source couplé audit autre noeud de la seconde charge ; et
une seconde source de courant constant (58) couplée entre le second potentiel de référence et à la fois un noeud de drain du troisième transistor MOS et un noeud de drain du quatrième transistor MOS,
dans lequel les premier et quatrième transistors MOS possèdent leurs noeuds de grille couplés l'un à l'autre, et les second et troisième transistors MOS possèdent leurs noeuds de grille couplés l'un à l'autre, les premier et second transistors MOS étant d'un premier type de conduction, et les troisième et quatrième transistors MOS étant d'un second type de conduction.

2. Amplificateur différentiel selon la revendication 1, dans lequel les premier et second transistors MOS sont des transistors à canal N, et les troisième et quatrième transistors MOS sont des transistors à canal P.

3. Amplificateur différentiel selon la revendication 1, dans lequel les premier et second transistors MOS sont des transistors à canal P, et les troisième et quatrième transistors MOS sont des transistors à canal N.

4. Amplificateur différentiel selon la revendication 1, dans lequel la première charge est un cinquième transistor MOS (54A), et la second charge est un sixième transistor MOS (55A).

5. Amplificateur différentiel selon la revendication 4, dans lequel un noeud de grille du cinquième transistor MOS et un noeud de grille du sixième transistor MOS sont couplés à un potentiel de polarisation commun (VBIAS1).

6. Amplificateur différentiel selon la revendication 1, dans lequel la première source de courant constant est un septième transistor MOS (53A), et la seconde source de courant constant est un huitième transistor MOS (58A).

7. Amplificateur différentiel selon la revendication 6, dans lequel un noeud de grille du septième transistor MOS et un noeud de grille du huitième transistor MOS sont couplés à un potentiel de polarisation commun.
